# Europäisches Patentamt

## European Patent Office

## Office européen des brevets

(11) Publication number: **0 218 825**
**B1**

(12) **EUROPEAN PATENT SPECIFICATION**

(45) Date of publication of patent specification: **20.09.89**

(51) Int. Cl.⁴: **C 12 N 15/00, C 12 P 21/02, A 61 K 45/02, C 12 P 19/34**

(21) Application number: **86110595.5**

(22) Date of filing: **13.10.83**

(80) Publication number of the earlier application in accordance with Art. 76 EPC: **0 109 748**

(54) Cysteine-depleted muteins of interferon-beta, their preparation, formulations containing them, and structural genes, vectors and organisms, and their production, suitable for use in the preparation of said muteins.

(30) Priority: **19.10.82 US 435154**
**15.04.83 US 486162**

(43) Date of publication of application:
**22.04.87 Bulletin 87/17**

(45) Publication of the grant of the patent:
**20.09.89 Bulletin 89/38**

(84) Designated Contracting States:
**AT BE CH DE FR GB IT LI LU NL SE**

(56) References cited:
**EP-A-0 041 313**

**NATURE, vol. 294, no. 5841, December 10, 1981, New York, USA. H.M. SHEPARD et al.: "A single amino acid change in INF-B1 abolishes its antiviral activity", pages 563-565**

(73) Proprietor: **CETUS CORPORATION**
**1400 Fifty-Third Street**
**Emeryville California 94608 (US)**

(72) Inventor: **Mark, David F.**
**217 Standbridge Ct.**
**Danville California 94526 (US)**
Inventor: **Lin, Leo S.**
**40880 Los Pines**
**Fremont California 94539 (US)**
Inventor: **Yu Lu, Shi-Da**
**19875 Lindenbrook Lane**
**Cupertino California 95014 (US)**

(74) Representative: **Bizley, Richard Edward et al**
**BOULT, WADE & TENNANT 27 Furnival Street**
**London EC4A 1PQ (GB)**

## Description

This invention is in the general area of recombinant DNA technology. More specifically it relates to mutationally altered biologically active Interferon-beta proteins that differ from their parent analogs by substitution/deletion of cysteine residues.

Biologically active proteins that are microbially produced via recombinant DNA (rDNA) technology may contain cysteine residues that are nonessential to their activity but are free to form undesirable intermolecular or intramolecular links. One such protein is microbially produced human beta interferon (IFN-β). In the course of the preparation of IFN-β by rDNA techniques, it has been observed that dimers and oligomers of microbially produced IFN-β are formed in *E. coli* extracts containing high concentrations of IFN-β. This multimer formation renders purification and separation of IFN-β very laborious and time-consuming and necessitates several additional steps in purification and isolation procedures such as reducing the protein during purification and reoxidizing it to restore it to its original conformation, thereby increasing the possibility of incorrect disulfide bond formation. Furthermore, microbially produced IFN-β has also been found to exhibit consistently low specific activity due perhaps to the formation of multimers or of random intramolecular disulfide bridges. It would be desirable, therefore, to be able to alter microbially produced biologically active proteins such as IFN-β in a manner that does not affect their activity adversely but reduces or eliminates their ability to form intermolecular crosslinks or intramolecular bonds that cause the protein to adopt an undesirable tertiary structure (e.g., a conformation that reduces the activity of the protein).

The present invention is directed to producing by directed mutagenesis techniques mutationally altered biologically active IFN-β proteins (such proteins are called "muteins", *Glossary of Genetics and Cytogenetics*, 4th Ed. p 381, Springer-Verlag (1976)) that retain the activity of their parent analogs but lack the ability to form intermolecular links or undesirable intramolecular disulfide bonds. In this regard Shepard, H. M., et al, *Nature* (1981) *294*: 563—565 describe a mutein of IFN-β in which the cysteine at position 141 of its amino acid sequence (there are three cysteines in native human IFN-β at positions 17, 31, and 141, *Gene* (1980) *10*:11—15 and *Nature* (1980) *285*:542—547) is replaced by tyrosine. This mutein was made by bacterial expression of a hybrid gene constructed from a partial IFN-β cDNA clone having a G→A transition at nucleotide 485 of the IFN-β gene. The mutein lacked the biological activity of native IFN-β leading the authors to conclude that the replaced cysteine was essential to activity.

Directed mutagenesis techniques are well known and have been reviewed by Lather, R. F. and Lecoq, J. P. in *Genetic Engineering* Academic Press (1983) pp 31—50. Oligonucleotide-directed mutagenesis is specifically reviewed by Smith, M. and Gillam, S. in *Genetic Engineering: Principles and Methods,* Plenum Press (1981) *3*:1—32.

The invention provides a biologically active recombinant synthetic interferon-beta mutein in which the cysteine residue normally present at position 17, numbered in accordance with nature human inteferon-beta, and which is free to form a disulfide link and is non-essential to said biological activity has been deleted or replaced by another amino acid.

Another aspect of the invention are synthetic structural genes having DNA sequences that have been specifically designed ("designer genes") to encode the above described synthetic muteins. Other aspects of this aspect are expression vectors that include such structural designer genes, host cells or organisms transformed with such vectors, and processes for making the synthetic mutein by culturing such transformants or their progeny and recovering the mutein from the culture. Therapeutic formulations whether for human or veterinary use (and optionally in unit dosage form) are another aspect of the invention.

Another aspect of the invention is a method of preventing a protein having one or more cysteine residues that is free to form an undesirable disulfide link from forming such a link characterized in that the protein is mutationally altered by deleting the cysteine residue(s) or replacing it/ them with other amino acids.

Still another aspect of the invention is a method for making the above described synthetic structural gene by oligonucleotide-directed mutagenesis comprising the following steps:

(a) hybridizing single-stranded DNA comprising a strand of a structural gene that encodes the parent protein with a mutant oligonucleotide primer that is complementary to a region of the strand that includes the codon for the cysteine to be deleted or replaced or the antisense triplet paired with the codon, as the case may be, except for a mismatch with that codon or antisense triplet, as the case may be, that defines a deletion of the codon or a triplet that encodes said other amino acid;

(b) extending the primer with DNA polymerase to form a mutational heteroduplex; and

(c) replicating the mutational heteroduplex.

The mutant oligonucleotide primers used in this process are another aspect of the invention.

Reference is made to copending European Patent Application No. 85109295.7 (EP—A—192811), a divisional application from the present application, which claims the general principle of a recombinant synthetic mutein of a biologically active protein, normally having at least one cysteine residue that is free to form a disulfide link and is non-essential to said biological activity in which at least one of said cysteine residues has been deleted or replaced by another amino acid.

Reference is also made to the European Patent EP—B—0136489 (published on 25.11.87) which discloses a number of possible amino acid substitutions in human interleukin-2, among them also the replacement of the cysteine residue in position 125 with alanine or serine. The European Patent EP—B—109748 relates to pharmaceutical and veterinary preparations of cysteine-125-depleted muteins of human interleukin-2.

The invention will now be further described and illustrated with reference to the accompanying drawings, in which:—

Figure 1 is a diagram of the amino acid sequence of IFN-β;

Figure 2 is a schematic illustration showing the preparation of a mutant IFN-β gene by oligonucleotide-directed mutagenesis;

Figure 3 shows a diagram of plasmid pβltrp including the IFN-β gene;

Figure 4 is a diagram of the cloning vector M13mp8 phage;

Figure 5 shows the restriction map of clone M13-β1;

Figure 6 shows the sequencing gel pattern of the mutant IFN-$\beta_{ser17}$ gene showing a single base change in the coding region;

Figure 7 is a diagram of the expression plasmid pTrp3;

Figure 8a shows the HinfI restriction pattern of clone pSY2501 and Figure 8b shows the resulting two 169 bp and 28 bp fragments thereof;

Figure 9 is a restriction map of clone pSY2501;

Figure 10 shows the coding DNA sequence for the mutein IFN-$\beta_{ser17}$ with the corresponding amino acid sequence therefor;

Figure 11 shows the single 18,000 dalton protein band corresponding to IFN-$\beta_{ser17}$ in the extracts of clones pSY2501 and pβltrp.

In the case of IFN-β, it has been reported in the literature and that both the glycosylated and unglycosylated IFNs show qualitatively similar specific activities and that, therefore, the glycosyl moieties are not involved in and do not contribute to the biological activity of IFN-β. However, bacterially produced IFN-β which is unglycosylated consistently exhibits quantitatively lower specific activity than native IFN-β which is glycosylated. IFN-β is known to have three cysteine residues at positions 17, 31 and 141. Cysteine 141 has been demonstrated by Shepard, et al, supra, to be essential for biological activity. In IFN-α, which contains four cysteine residues, there are two intramolecular —S—S— bonds: one between cys 29 and cys 138 and another between cys 1 and cys 98. Based on the homology between IFN-β and IFN-αs cys 141 of IFN-β could be involved in an intramolecular —S—S— bond with cys 31, leaving cys 17 free to form intermolecular crosslinks. By either deleting cys 17 or substituting it by a different amino acid, one can determine whether cys 17 is essential to biological activity, and its role in —SS— bond formation. If cys 17 is not essential for the biological activity of the protein, the resulting cys 17-deleted or cys 17-substituted protein might exhibit specific activity close to that of native IFN-β and would possibly also facilitate isolation and purification of the protein.

By the use of the oligonucleotide-directed mutagenesis procedure with a synthetic oligonucleotide primer that is complementary to the region of the IFN-β gene at the codon for cys 17 but which contains single or multiple base changes in that codon, a designer gene may be produced that results in cys 17 being replaced with any other amino acid of choice. When deletion is desired the oligonucleotide primer lacks the codon for cys 17. Conversion of cys 17 to neutral amino acids such as glycine, valine, alanine, leucine, isoleucine, tyrosine, phenylalanine, histidine, tryptophan, serine, threonine and methionine is the preferred approach. Serine and threonone are the most preferred replacements because of their chemical analogy to cysteine. When the cysteine is deleted, the mature mutein is one amino acid shorter than the native parent protein or the microbially produced IFN-β.

The size of the oligonucleotide primer is determined by the requirement for stable hybridization of the primer to the region of the gene in which the mutation is to be induced, and by the limitations of the currently available methods for synthesizing oligonucleotides. The factors to be considered in designing oligonucleotides for use in oligonucleotide-directed mutagenesis (e.g., overall size, size of portions flanking the mutation site) are described by Smith, M. and Gillam S., supra. In general the overall length of the oligonucleotide will be such as to optimize stable, unique hybridization at the mutation site with the 5' and 3' extensions from the mutation site being of sufficient size to avoid editing of the mutation by the exonuclease activity of the DNA polymerase. Oligonuclotides used for mutagenesis in accordance with the present invention usually contain from about 12 to about 24 bases, preferably from about 14 to about 20 bases and still more preferably from about 15 to about 18 bases. They will usually contain at least about three bases 3' of the altered or missing codon.

The method for preparing the modified IFN-β gene broadly involves inducing a site-specific mutagenesis in the IFN-β gene at codon 17 (TGT) using a synthetic nucleotide primer which omits the codon or alters it so that it codes for another amino acid. When threonine replaces the cysteine and the primer is hybridized to the antisense strand of the IFN-β gene, the preferred nucleotide primer is

GCAATTTTCAG<u>ACT</u>CAG

(underlining denotes the altered codon). When it is desirable to delete cysteine, the preferred primer is

AGCAATTTTCAGCAGAAGCTCCTG,

which omits the TGT codon for cys. When cysteine is replaced by serine, a 17-nucleotide primer,

GCAATTTTCAG<u>AGT</u>CAG,

which includes an AGT codon for serine is the primer of choice. The T→A transition of the first base in the cys 17 codon results in changing cysteine to serine. It must be recognized that when deletions are introduced, the proper reading frame for the DNA sequence must be maintained for expression of the desired protein.

The primer is hybridized to single-stranded phage such as M13, fd, or φX174 into which a strand of the IFN-β gene has been cloned. It will be appreciated that the phage may carry either the sense strand or antisense strand of the gene. When the phage carries the antisense strand the primer is identical to the region of the sense strand that contains the codon to be mutated except for a mismatch with that codon that defines a deletion of the codon or a triplet that codes for another amino acid. When the phage carries the sense strand the primer is complementary to the region of the sense strand that contains the codon to be mutated except for an appropriate mismatch in the triplet that is paired with the codon to be deleted. Conditions that may be used in the hybridization are described by Smith M. and Gillam, S., supra. The temperature will usually range between about 0°C and 70°C, more usually about 10°C to 50°C. After the hybridization, the primer is extended on the phage DNA by reaction with DNA polymerase I, T₄ DNA polymerase, reverse transcriptase or other suitable DNA polymerase. The resulting dsDNA is converted to closed circular dsDNA by treatment with a DNA ligase such as T₄ DNA ligase. DNA molecules containing single-stranded regions may be destroyed by S1 endonuclease treatment.

The resulting mutational heteroduplex is then used to transform a competent host organism or cell. Replication of the heteroduplex by the host provides progeny from both strands. Following replication the mutant gene may be isolated from progeny of the mutant strand, inserted into an appropriate expression vector, and the vector used to transform a suitable host organism or cell. Preferred vectors are plasmids pBR322, pCR1, and variants thereof, synthetic vectors and the like. Suitable host organisms are *E. coli* yeast, animal cells such as mice, rat or Chinese hamster ovary (CHO) cells, and the like. It must be recognized that when a host of choice is transformed with the vector, appropriate promoter-operator sequences are also introduced in order for the mutein to be expressed. Hosts may be prokaryotic or eukaryotic (processes for inserting DNA into eukaryotic cells are described in PCT applications Nos US81/00239 and US81/00240 published 3 September 1981). *E. coli* and CHO cells are the preferred hosts. The muteins obtained in accordance with the present invention may be glycosylated or unglycosylated depending on the glycosylation occurring in the native parent protein and the host organism used to produce the mutein. If desired, unglycosylated mutein obtained when *E. coli* or a *Bacillus* is the host organism, may be optionally glycosylated *in vitro* by chemical, enzymatic and other types of modifications known in the art.

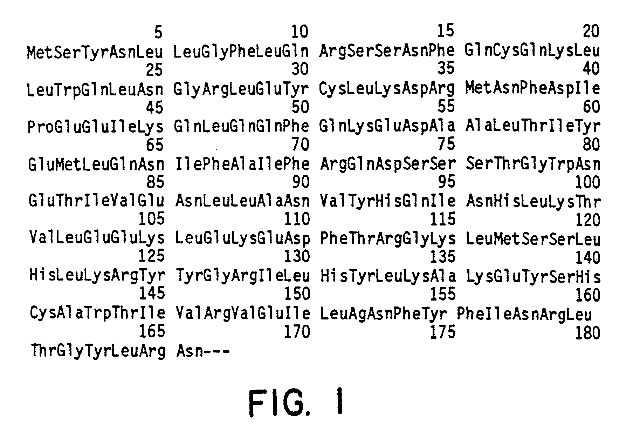

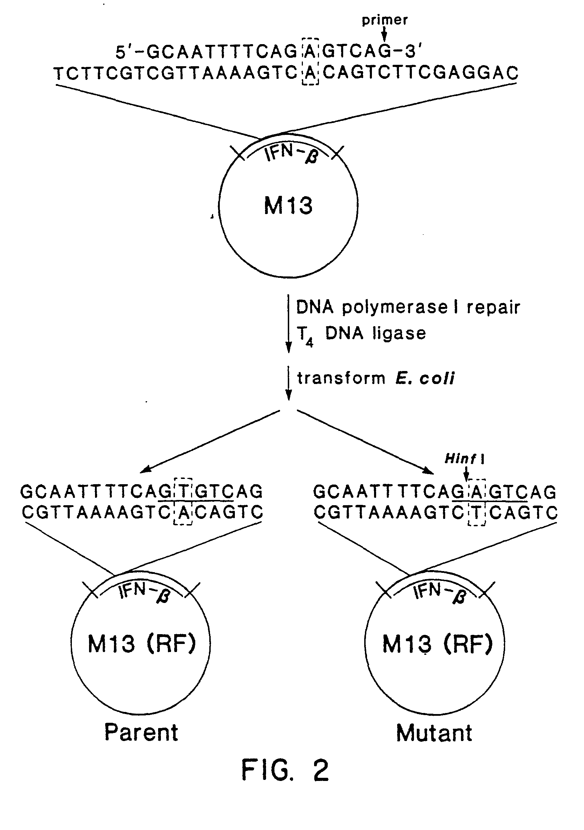

In IFN-β, the cysteine residue at positions 17 in the amino acid sequence of IFN-β, as shown in Figure 1, is changed to serine by a T→A transition of the first base of codon 17 of the sense strand of the DNA sequence which codes for the mature IFN-β. The site-specific mutagensis is induced using a synthetic 17-nucleotide primer

GCAATTTTCAGAGTCAG

which is identical to a seventeen nucleotide sequence on the sense strand of IFN-β in the region of codon 17 except for a single base mismatch at the first base of codon 17. The mismatch is at nucleotide 12 in the primer. It must be recognized that the genetic code is degenerate and that many of the amino acids may be encoded by more than one codon. The base code for serine, for example, is six-way degenerate such that the codons, TCT, TCG, TCC, TCA, AGT, and ACG all code for serine. The AGT codon was chosen for the preferred embodiment for convenience. Similarly, threonine is encoded by any one of codons ACT, ACA, ACC and ACG. It is intended that when one codon is specified for a particular amino acid, it includes all degenerate codons which encode that amino acid. The 17-mer is hybridized to single-stranded M13 phage DNA which carries the antisense strand of the IFN-β gene. The oligonucleotide primer is then extended on the DNA using DNA polymerase I Klenow fragment and the resulting dsDNA is converted to closed circular DNA with T₄ ligase. Replication of the resulting mutational heteroduplex yields clones from the DNA strand containing the mismatch. Mutant clones may be identified and screened by the appearance or disappearance of specific restriction sites, antibiotic resistance or sensitivity, or by other methods known in the art. When cysteine is substituted with serine, the T→A transition, shown in Figure 2, results in the creation of a new *Hin*fI restriction site in the structural gene. The mutant clone is identified by using the oligonucleotide primer as a probe in a hybridization screening of the mutated phage plaques. The primer will have a single mismatch when hybridized to the parent but will have a perfect match when hybridized to the mutated phage DNA, as indicated in Figure 2. Hybridization conditions can then be devised where the oligonucleotide primer will preferentially hybridize to the mutated DNA but not to the parent DNA. The newly generated *Hin*fI site also serves as a means of confirming the single base mutation in the IFN-β gene.

The M13 phage DNA carrying the mutated gene is isolated and spliced into an appropriate expression vector, such as plasmid pTrp3, and *E. coli* strain MM294 is transformed with the vector. Suitable growth media for culturing the transformants and their progeny are known to those skilled in the art. The expressed mutein of IFN-β is isolated, purified and characterized.

The following Examples are presented to help in the better understanding of the subject invention and for purposes of illustration only. They are not to be construed as limiting the scope of the invention.

Example 1
Cloning of the IFN-β gene into M13 vector:

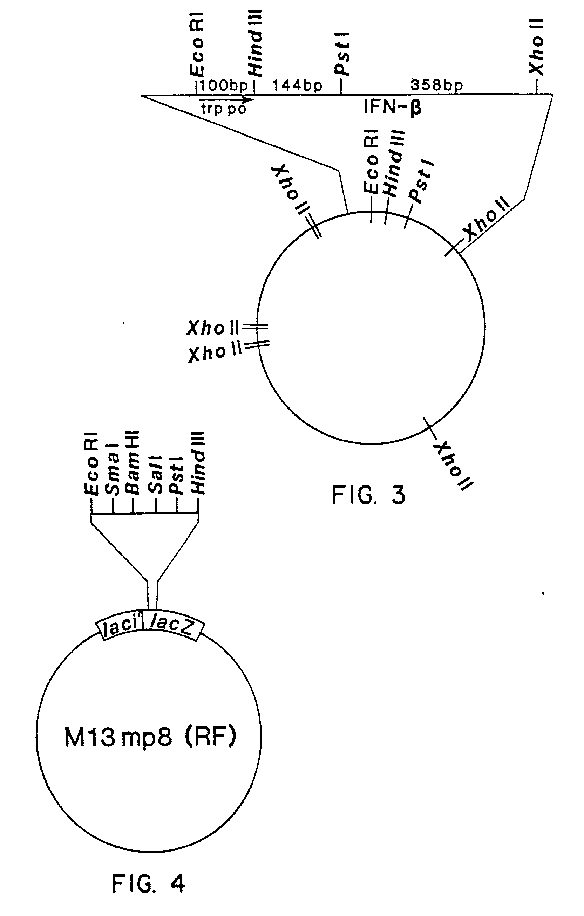

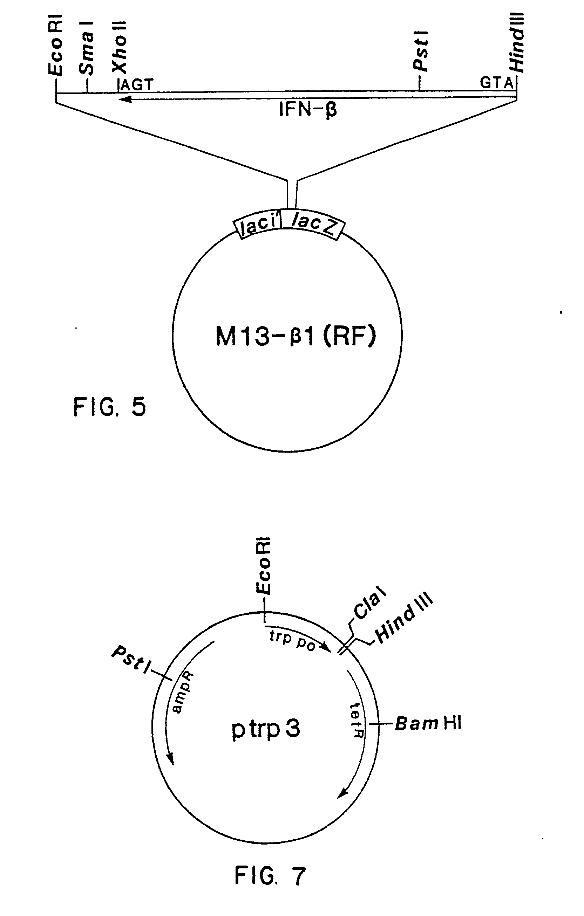

The use of M13 phage vector as a source of single-stranded DNA template has been demonstrated by G. F. Temple et al, *Nature* (1982) *296*:537—540. Plasmid pβltrp (Figure 3) containing the IFN-β gene under control of *E. coli* trp promoter, was digested with the restriction enzymes *Hind*III and *Xho*II. The M13mp8 (J. Messing, "Third Cleveland Symposium on Macromolecules: Recombinant DNA," Ed. A. Walton, Elsevier Press, 143—153 (1981)) replicative form (RF) DNA (Figure 4) was digested with restriction enzymes *Hind*III and *Bam*HI, and mixed with the pβltrp DNA which had previously been digested with *Hind*III and *Xho*II. The mixture was then ligated with T₄ DNA ligase and the ligated DNA transformed into competent cells of *E. coli* strain JM 103 and plated on Xgal indicator plates (J. Messing, et al, *Nucleic Acids Res* (1981) *9*:309—321). Plaques containing recombinant phage (white plaques) were picked, inoculated into a fresh culture of JM 103 and minipreps of RF molecules prepared from the infected cells (H. D. Birnboim and J. Doly, *Nucleic Acid Res* (1979) *7*:1513—1523). The RF molecules were digested with various restriction enzymes to identify the clones containing the IFN-β insert. The restriction map of one such clone (M13-β1) is shown in Figure 5. Single-stranded (ss) phage DNA was prepared from clone M13-β1 to serve as a template for site-specific mutagensis using a synthetic oligonucleotide.

Example 2
Site-specific mutagenesis:

Forty picomoles of the synthetic oligonucleotide

GCAATTTTCAGAGTCAG

(primer) was treated with T₄ kinase in the presence of 0.1 mM adenosine triphosphate (ATP), 50 mM hydroxymethylaminomethane hydrochloride (Tris-HCl) pH 8.0, 10 mM MgCl₂, 5 mM dithiothreitol (DTT) and 9 units of T₄ kinase, in 50 μl at 37°C for 1 hr. The kinased primer (12 pmole) was hybridized to 5 μg of ss M13-β1 DNA in 50 μl of a mixture containing 50 mM NaCl, 10 mM Tris-HCl, pH 8.0, 10 mM MgCl₂ and 10 mM β-mercaptoethanol, by heating at 67°C for 5 min and at 42°C for 25 min. The annealed mixture was then chilled on ice and then added to 50 μl of a reaction mixture containing 0.5 mM each of deoxynucleoside triphosphate (dNTP), 80 mM Tris-HCl, pH 7.4, 8 mM MgCl₂, 100 mM NaCl, 9 units of DNA polymerase I, Klenow fragment, 0.5 mM ATP and 2 units of T₄ DNA ligase, incubated at 37°C for 3 hr and at 25°C for 2 hr. The reaction was then terminated by phenol extraction and ethanol precipitation. The DNA was dissolved in 10 mM Tris-HCl pH 8.0, 10 mM ethylenediaminetetraacetic acid (EDTA), 50% sucrose and 0.05% bromophenyl blue and electrophoresed on 0.8% agarose gel in the presence of 2 μg/ml of ethidium bromide. The DNA bands corresponding to the RF forms of M13-β1 were eluted from gel slices by the perchlorate method (R. W. Davis, et al, "Advanced Bacterial Genetics", Cold Spring Harbor Laboratory, N. Y., p. 178—179 (1980)). The eluted DNA was used to transform competent JM 103 cells, grown overnight and ssDNA isolated from the culture supernatant. This ssDNA was used as a template in a second cycle of primer extension, the gel purified RF forms of the DNA were transformed into competent JM 103 cells, plated onto agar plates and incubated overnight to obtain phage plaques.

Example 3
Site specific mutagenesis:

The experiment of Example 2 above is repeated except that the synthetic oligonucleotide primer used is

GCAATTTTCAGACTCAG

to change codon 17 of the IFN-β gene from one that codes for cysteine to one that codes for threonine.

Example 4
Site specific deletion

The experiment of Example 2 above is repeated except that the synthetic oligonucleotide primer used is

AGCAATTTTCAGCAGAAGCTCCTG

to delete codon 17 of the IFN-β gene.

Example 5
Screening and identification of mutagenized plaques:

Plates containing mutated M13-β1 plaques (Example 1) as well as two plates containing unmutated M13-β1 phage plaques, were chilled to 4°C and plaques from each plate transferred onto two nitrocellulose filter circles by layering a dry filter on the agar plate for 5 min for the first filter and 15 min for the second filter. The filters were then placed on thick filter papers soaked in 0.2 N NaOH, 1.5 M NaCl and 0.2% Triton X-100 for 5 min, and neutralized by layering onto filter papers soaked with 0.5 M tris-HCl, pH 7.5 and 1.5 M NaCl for another 5 min. The filters were washed in a similar fashion twice on filters soaked in 2×SSC (standard saline citrate), dried and then baked in a vacuum oven at 80°C for 2 hr. The duplicate filters were prehybridized at 55°C for 4 hr with 10 ml per filter of DNA hybridization buffer (5×SSC) pH 7.0, 4×Denhardt's solution (polyvinylpyrrolidine, ficoll and bovine serum albumin,

1×=0.02% of each), 0.1% sodium dodecyl sulfate (SDS), 50 mM sodium phosphate buffer pH 7.0 and 100 µg/ml of denatured salmon sperm DNA. $^{32}$P-labeled probe was prepared by kinasing the oligonucleotide primer with $^{32}$P-labeled ATP. The filters were hybridized to $3.5\times10^5$ cpm/ml of $^{32}$P-labeled primer in 5 ml per filter of DNA hybridization buffer at 55°C for 24 hr. The filters were washed at 55°C for 30 min each in washing buffers containing 0.1% SDS and decresing amounts of SSC. The filters were washed initially with buffer containing 2×SSC and the control filters containing unmutated M13-β1 plaques were checked for the presence of any radioactivity using a Geiger counter. The concentration of SSC was lowered stepwise and the filters washed until no detectable radioactivity remained on the control filters with the unmutated M13-β1 plaques. The lowest concentration of SSC used was 0.1×SSC. The filters were air dried and autoradiographed at −70°C for 2—3 days. 480 plaques of mutated M13-β1 and 100 unmutated control plaques were screened with the kinased oligonucleotide probe. None of the control plaques hybridized with the probe while 5 mutated M13-β1 plaques hybridized with the probe.

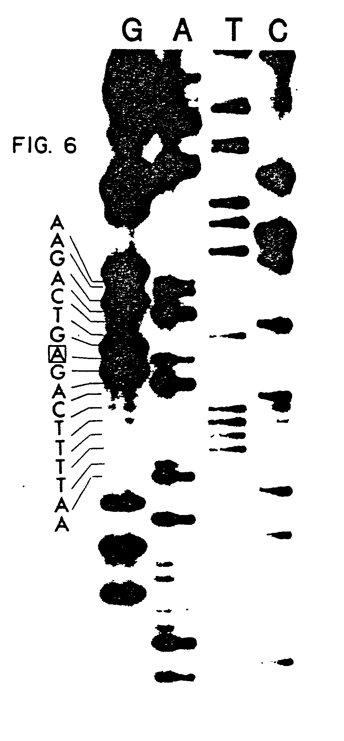

One of the five mutated M13-β1 plaques (M13-SY2501) was picked and inoculated into a culture of JM 103. ssDNA was prepared from the supernatant and double-stranded (ds) DNA was prepared from the cell pellet. The ssDNA was used as a template for the dideoxy-sequencing of the clone using the M13 universal primer. The result of the sequence analysis is shown in Figure 6, confirming that the TGT cys codon has been converted to an AGT ser codon.

Example 6
Expression of mutated IFN-β in *E. coli*

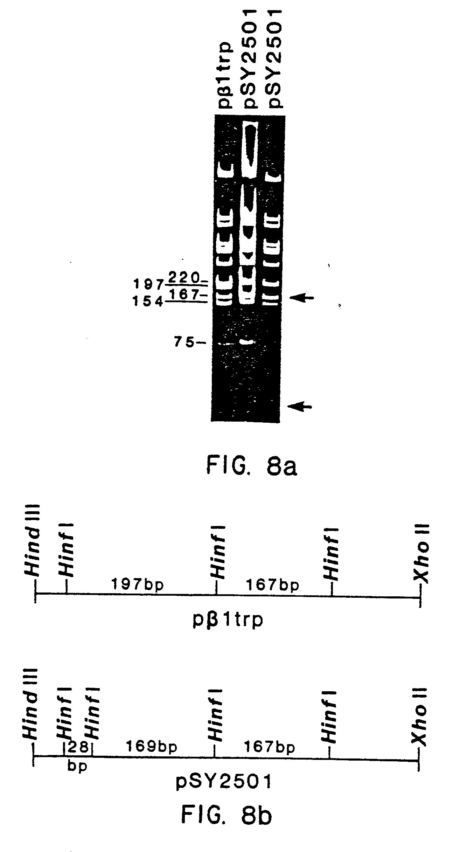

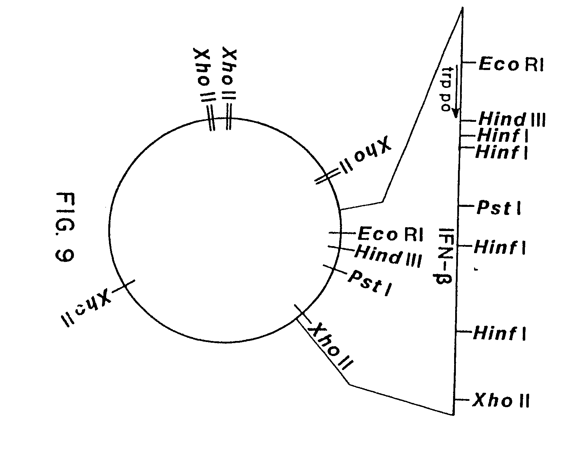

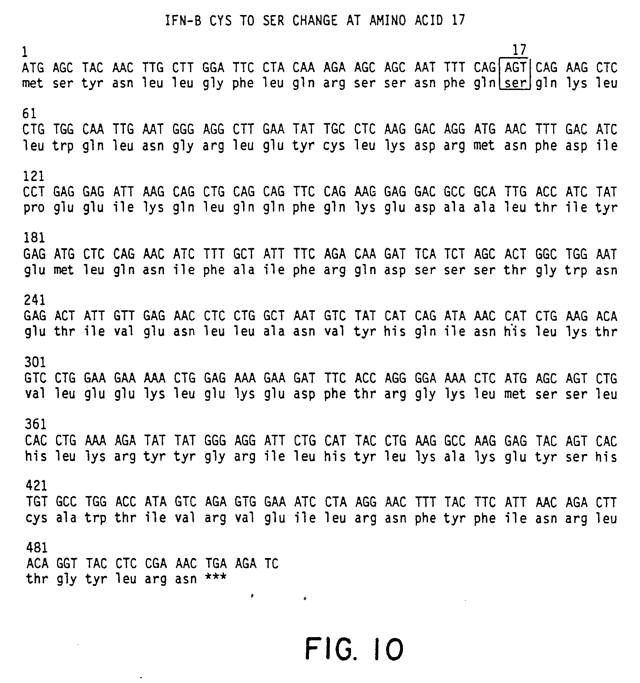

RF DNA from M13-SY2501 was digested with restriction enzymes *Hind*III and *Xho*II and the 520 bp insert fragment purified on a 1% agarose gel. The plasmid pTrp3 containing the *E. coli* trp promoter (Figure 7) was digested with the enzymes *Hind*III and *Bam*HI, mixed with the purified M13-SY2501 DNA fragment, and ligated in the presence of T$_4$ DNA ligase. The ligated DNA was transformed into *E. coli* strain MM294. Ampicillin resistant transformants were screened for sensitivity to the drug tetracycline. Plasmid DNA from five ampicillin resistant, tetracycline sensitive clones were digested with *Hin*fI to screen for the presence of the M13-SY2501 insert. Figure 8a shows the *Hin*fI restriction pattern of one of the clones (pSY2501) comparing it with the *Hin*fI pattern of the original IFN-β clone, pβltrp. As expected, there is an additional *Hin*fI site in pSY2501, cleaving the 197 bp IFN-β internal fragment to a 169 bp fragment and a 28 bp fragment (Figure 8b). A restriction map of the clone pSY2501 is shown in Figure 9. The complete DNA sequence of the mutant IFN-β gene is shown in Figure 10 together with the predicted amino acid sequence.

The plasmid designated as clone pSY2501 was deposited on 30 March 1933 with the Agricultural Research Culture Collection (NRRL), Fermentation Laboratory, Northern Regional Research Center, Science and Education Administration, U.S. Department of Agriculture, 1815 North University Street, Peoria, Illinois 60604 and is assigned accession numbers CMCC No. 1533 and NRRL No. B-15356.

Cultures of pSY2501 and pβltrp, which include progeny thereof, were grown up to an optical density (OD$_{600}$) of 1.0. Cell free extracts were prepared and the amount of IFN-β antiviral activity assayed on GM2767 cells in a microtiter assay. Extracts of clone pSY2501 exhibited three to ten times higher activity than pβltrp (Table I), indicating that clone pSY2501 was either synthesizing more protein exhibiting IFN-β activity or that the protein made had a higher specific activity.

TABLE I

| Extract | Antiviral activity (U/ml) |
|---|---|
| pSY2501 | $6\times10^5$ |
| pβltrp | $1\times10^5$ |
| ptrp3 (control) | 30 |

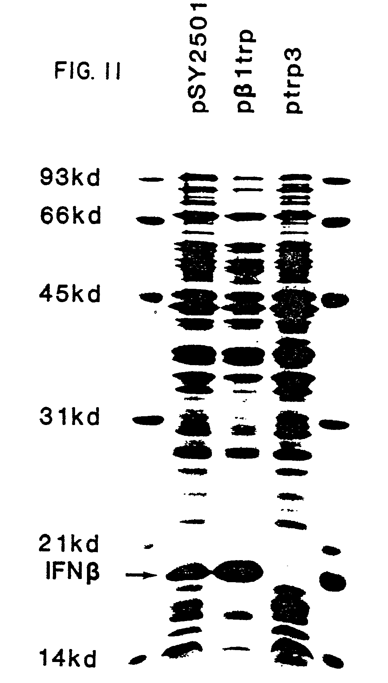

In order to determine if clone pSY2501 was synthesizing several times more active protein, the extracts of both clones were electrophoresed on a SDS polyacrylamide gel together with a control extract and the gel stained with coomasie blue to visualize the proteins. As shown in Figure 11, there was only one protein band corresponding to an apparent 18,000 dalton protein that was present in the extracts of clones pSY2501 and pβltrp but not in the control extract of ptrp3. This protein, which has a molecular weight of about 20,000 daltons but shows a gel migration pattern of an 18,000 dalton protein was previously shown to be IFN-β by purification of this protein from extracts of pβltrp. Since there is less of this protein in extracts of pSY2501 than in extracts of pβltrp, the specific activity of the protein in extracts of clone pSY2501 was higher than that of clone pβltrp.

Example 7
Purification of IFN-β$_{ser17}$:

IFN-β$_{ser17}$ was recovered from *E. coli* that had been transformed to produce IFN-β$_{ser17}$. The *E. coli* were grown in the following growth medium to an OD of 10—11 at 680 nm (dry wt. 8.4 g/l).

| Ingredient | Concentration |
|---|---|
| $NH_4Cl$ | 20 mM |
| $K_2SO_4$ | 16.1 mM |
| $KH_2PO_4$ | 7.8 mM |
| $Na_2HPO_4$ | 12.2 mM |
| $MgSO_4 \cdot 7H_2O$ | 3 mM |
| $Na_3$ citrate $\cdot 2H_2O$ | 1.5 mM |
| $MnSO_4 \cdot 4H_2O$ | 30 µM |
| $ZnSO_4 \cdot 7H_2O$ | 30 µM |
| $CuSO_4 \cdot 5H_2O$ | 3 µM |
| L-tryptophan | 70 mg/l |
| $FeSO_4 \cdot 7H_2O$ | 72 µM |
| thiamine $\cdot$ HCl | 20 mg/l |
| glucose | 40 g/l |

pH control with $NH_4OH$

A 9.9 l (9.9 kg) harvest of the transformed *E. coli* was cooled to 20°C and concentrated by passing the harvest through a cross-flow filter at an average pressure drop of ~110 kpa and steady-state filtrate flow rate of 260 ml/min until the filtrate weight was 8.8 kg. The concentrate (approximately one liter) was drained into a vessel and cooled to 15°C. The cells in the concentrate were then disrupted by passing the concentrate through a Manton-Gaulin homogenizer at 5°C, ~69,000 kpa. The homogenizer was washed with one liter phosphate buffered saline, pH 7.4 (PBS), and the wash was added to the disruptate to give a final volume of two liters. This volume was continuously centrifuged at 12000×g at a 50 ml/min flow rate. The solid was separated from the supernatant and resuspended in four liters PBS containing 2% by wt. SDS. This suspension was stirred at room temperature for 15 min after which there was no visible suspended material. The solution was then extracted with 2-butanol at a 1:1 2-butanol:solution volume ratio. The extraction was carried out in a liquid-liquid phase separator using a flow rate of 200 ml/min. The organic phase was then separated and evpaorated to dryness to yield 21.3 g of protein. This was resuspended in distilled water at a 1:10 volume ratio.

The recovered product was assayed for human IFN-β activity using an assay based on protection against viral cytopathic effect (CPE). The assay was made in microtiter plates. Fifty µl of minimum essential medium were charged into each well and 25 µl of the sample was placed in the first well and 1:3 volume dilutions were made serially into the following wells. Virus (vesicular stomatitis), cell (human fibroblast line GM-2767), and reference IFN-β controls were included on each plate. The reference IFN-β used was 100 units per ml. The plates were then irradiated with UV light for 10 min. After irradiation 100 µl of the cell suspension ($1.2×10^5$ cells/ml) was added to each well and the trays were incubated for 18—24 hr. A virus solution at one plaque-forming unit per cell was added to each well except the cell control. The trays were then incubated until the virus control showed 100% CPE. This normally occurred 18—24 hr after adding the virus solution.

Assay results were interpreted in relation to the location of the 50% CPE well of the reference IFN-β control. From this point the titer of interferon for all samples on the plate was determined. The specific activity of the recovered product was determined to be $5×10^7$ U/mg.

Example 8
Acid preciptation and chromatographic purification

The process of Example 7 was repeated except that after extraction and separation of the aqueous and organic phases and mixing of the organic phase with PBS at a volume ratio of 3:1 the pH of the mixture was lowered to about 5 by addition of glacial acetic acid. The resulting precipitate was separated by centrifugation at 10000—17000×g for 15 min and the pellet was redissolved in 10% w/v SDS, 10 mM DTT, 50 mM sodium acetate buffer, pH, 5.5, and heated to 80°C for 5 min.

The solution was then applied to a Brownlee RP-300, 10 µM, "Aquapore"® column using a Beckman gradient system. Buffer A was 0.1% trifluoroacetic acid (TFA) in $H_2O$; buffer B was 0.1% TFA in acetonitrile. Detection was by ultraviolet absorbance at 280 nm. The solvent program was linear gradient of 0% buffer B to 100% buffer B in three hr. Fractions containing highest interferon activities were pooled and the specific activity of the pooled interferon preparation was determined to be $9.0×10^7$ to $3.8×10^8$ international units per mg protein, as compared to about $2×10^8$ U/mg for native IFN-β.

Example 9
Biochemical characterization of IFN-β Ser$_{17}$

Amino acid compositions were determined after 24—72 hr timed hydrolysis of 40 µg samples of IFN in 200 µl of 5.7 N HCl, 0.1% phenol, at 108°C. Proline and cysteine were determined in the same fashion after performic acid oxidation; in this case, phenol was omitted from the hydrolysis. Tryptophan was analyzed after 24 hr hydrolysis of 400 µl samples in 5.7 N HCl, 10% mercaptoacetic acid (no phenol). Analysis was performed on a Beckman 121MB amino acid analyzer using a single column of AA10 resin.

The amio acid composition calculated from representative 24-, 48-, 72-hr acid hydrolyses of purified IFN-β Ser$_{17}$ agrees well with that predicted by the DNA sequence of the clones IFN gene, minus the missing N-terminal methionine.

The amino acid sequence of the first 58 residues from the amino acid terminus of purified IFN was determined on a 0.7 mg sample in a Beckman 890C sequenator with 0.1 M Quadrol® buffer. PTH amino acids were determined by reverse-phase HPLC on an Altex ultrasphere ODS column (4.6×250 mm) at 45°C eluted at 1.3 min at 40% buffer B, and 8.4 min from 40—70% buffer B, where buffer A was 0.0115 M sodium acetate, 5% tetrahydrofuran (THF), pH 5.11 and buffer B was 10% THF in acetonitrile.

The N-terminal amino acid sequence of IFN-β Ser$_{17}$ determined matches the expected sequence predicted from the DNA sequence, except for the absence of N-terminal methionine.

As indicated above, the IFN-$\beta_{ser17}$ preparation exhibits specific activity levels very close to or better than that of native IFN-$\beta$. IFN-$\beta_{ser17}$ has no free sulfhydryl groups but indicates one —S—S— bond between the only remaining cysteines at positions 31 and 141. The protein does not readily form oligomers and appears to be substantially in the monomeric form. The IFN-$\beta_{ser17}$ obtained in accordance with this invention may be formulated either as a single product or mixtures of the various forms, into pharmaceutically or veterinarily acceptable preprations, e.g. in inert, non-toxic, nonallergenic, physiologically compatible carrier media for clinical and therapeutic uses in cancer therapy or in conditions where interferon therapy is indicated and for viral infections. Such formulations may be in unit dosage form. Such media include but are not limited to distilled water, physiological saline, Ringer's solution, Hank's solution and the like. Other nontoxic stabilizing and solubilizing additives such as dextose, HSA (human serum albumin) and the like may be optimally included. The therapeutic formulations may be administered orally or parenterally such as intravenous, intramuscular, intraperitoneal and subcutaneous administrations. Preparations of the modified IFN-$\beta$ of the present invention may also be used for topical applications in appropriate media normally utilized for such purposes.

The principal advantages of the above described mutein of IFN-$\beta$ lie in the elimination of a free sulfhydryl group at position 17 in IFN-$\beta$, thereby forcing the protein to form correct disulfide links between cys 31 and cys 141 and to assume the conformation ostensibly required for full biological activity. The increased specific activity of the IFN-$\beta_{ser17}$ enables the use of smaller dosages in therapeutic uses. By deleting the cysteine at position 17 and eliminating the free —SH group, the IFN-$\beta_{ser17}$ protein does not form dimers and oligomers so readily as the microbially produced IFN-$\beta$. This facilitates purification of the protein and enhances its stability.

**Claims**

1. A biologically active recombinant synthetic human interferon-beta mutein in which the cysteine residue normally present at position 17, numbered in accordance with native human interferon-beta, and which is free to form a disulfide link and is non-essential to said biological activity has been deleted or replaced by a neutral amino acid.

2. A mutein as claimed in Claim 1 wherein said cysteine is replaced by serine, threonine, glycine, alanine, valine, leucine, isoleucine, histidine, tyrosine, phenylalanine, tryptophan or methionine.

3. A mutein as claimed in Claim 1, wherein said neutral amino acid is serine.

4. A human recombinant serine$_{17}$interferon-beta mutein.

5. A human recombinant serine$_{17}$interferon-beta mutein which exhibits the biological activity of native human interferon-beta and which has the deduced amino acid sequence as represented in Figure 10 with or without N-terminal methionine.

6. A mutein as claimed in any one of Claims 1 to 5 and which is unglycosylated.

7. A structural gene having a DNA sequence that encodes a synthetic mutein as defined in any one of Claims 1 to 6.

8. An expression vector that includes a structural gene as defined in Claim 7.

9. Plasmid pSY2501, as obtainable from the *E. coli* strain deposited under the accession nr. NRRL B-15356.

10. *E. coli* transformed with an expression vector as defined in Claim 8 or with a plasmid as defined in Claim 9.

11. The use of an expression vector as defined in Claim 8 or a plasmid as defined in Claim 9 in making a synthetic recombinant human interferon-beta mutein by expressing said vector or plasmid.

12. A method of preventing a human interferon-beta protein having at least one cysteine residue that is free to form an incorrect disulfide link from forming said link comprising mutationally altering the protein by deleting the cysteine residue at position 17, numbered in accordance with native human interferon-beta, or replacing the said cysteine residue with a neutral amino acid.

13. A method as claimed in Claim 12, wherein the cysteine residue is replaced with serine or threonine.

14. A method for making a structural gene as defined in Claim 7 comprising:

(a) a hybridizing single-stranded DNA comprising a strand of a structural gene that encodes for a human interferon-beta protein with a mutant oligonucleotide primer that is complementary to a region of said strand that includes the codon for said cysteine residue or the antisense triplet paired with said codon, as the case may be, except for a mismatch with said codon or said antisense triplet which mismatch defines a deletion of the codon or a triplet that codes for said neutral amino acid;

(b) extending the primer with DNA polymerase to form a mutational heteroduplex; and

(c) replicating said mutational heteroduplex.

15. A method as claimed in Claim 14, wherein the mismatch defines a triplet that codes for serine or threonine.

16. A method as claimed in Claim 14, or Claim 15, wherein the single-stranded DNA is a single-stranded phage that includes said strand and the mutational heteroduplex of step (b) is converted to a closed circular heteroduplex.

17. A method as claimed in any one of Claims 14 to 16, wherein said replicating is effected by transforming a competent bacterial host with the closed circular heteroduplex and culturing the resulting transformants.

18. A method as claimed in any one of Claims 14 to 17 and including the additional steps of isolating progeny of the mutant strand of the heteroduplex, isolating DNA from said progeny, and isolating said gene from the DNA from said progeny.

19. A method as claimed in any one of Claims 14 to 18, wherein the mismatch defines a codon that codes for serine.

20. An oligonucleotide for use in making a structural gene as defined in Claim 7 by oligonucleotide-directed mutagenesis and having a nucleotide sequence that is complementary to a region of the strand of the structural gene that includes the codon for the cysteine residue or the antisense triplet paired with said codon, as the case may be, except for a mismatch with said codon that defines a triplet that codes for said neutral amino acid.

21. A process for preparing an oligonucleotide suitable for use in making a structural gene as defined in Claim 7 by oligonucleotide-directed mutagenesis which comprises synthesizing by a manner known *per se* a nucleotide sequence that is complementary to a region of the stand of the structural gene that includes the codon for the cysteine residue or the antisense triplet paired with said codon, as the case may be, except for a mismatch with said codon that defines a deleting of the codon or a triplet that codes for a neutral amino acid.

22. A process for producing a vector as defined in Claim 8 comprising inserting into a vector capable of transforming a host cell a gene as defined in Claim 7 in a position permitting expression of said gene.

23. A pharmaceutical or veterinary formulation having human interferon-beta activity, comprising a synthetic mutein preparation as claimed in any one of Claims 1 to 6 formulated for pharmaceutical or veterinary use, respectively, and optionally also comprising a pharmaceutically or veterinarily acceptuble diluent, carrier or excipient and/or optionally being in unit dosage form.

24. A formulation for use in cancer therapy, comprising:
(a) an effective amount of a recombinant human interferon-beta mutein, wherein the cysteine residue at position 17, numbered in accordance with native human interferon-beta, is deleted or replaced by another amino acid an said mutein exhibits the biological activity of native, human interferon-beta; and
(b) a non-toxic, non-allergenic, therapeutically acceptable carrier medium.

25. A formulation as claimed in Claim 24, wherein the mutein is human interferon-beta-serine$_{17}$ and said carrier is distilled water, Ringer's solution, Hank's solution or physiological saline.

26. A formulation as claimed in Claim 24 or Claim 25 which includes non-toxic stabilizing and solubilizing additives selected from dextrose and human serum albumin.

27. A formulation as claimed in any one of Claims 24 to 26 and in unit dosage form.

28. A formulation as claimed in any one of Claims 24 to 26 and in topical application form.

29. A synthetic mutein as claimed in any one of Claims 1 to 6 for use in prophylaxis, therapy or diagnosis practised on the human or animal body.

30. A method for making a pharmaceutical or verterinary formulation as defined in Claim 23, which method comprises formulating for pharmaceutical or veterinary use, respectively, a synthetic mutein as defined in any one of Claims 1 to 6.

**Patentansprüche**

1. Biologisch aktives, rekombinantes, synthetisches menschliches β-Interferon-Mutein, in dem der entsprechend der Numerierung von natürlichem menschlichem β-Interferon normalerweise an Position 17 befindliche Cystein-Rest, der zu Bildung einer Disulfidbindung verfügbar und für die biologische Aktivität nicht essentiell ist, deletiert oder durch eine neutrale Aminosäure ersetzt worden ist.

2. Mutein nach Anspruch 1, in dem das Cystein durch Serin, Threonin, Glycin, Alanin, Valin, Leucin, Isoleucin, Histidin, Tyrosin, Phenylalanin, Tryptophan oder Methionin ersetzt ist.

3. Mutein nach Anspruch 1, in dem die neutrale Aminosäure Serin ist.

4. Menschliches rekombinantes Serin$_{17}$-β-Interferon-Mutein.

5. Menschliches rekombinantes Serin$_{17}$-β-Interferon-Mutein, das die biologische Aktivität von natürlichem, menschlichem β-Interferon aufweist und die in Figure 10 dargestellte, abgeleitete Aminosäure-Sequenz mit oder ohne N-terminalem Methionin aufweist.

6. Mutein nach einem der Ansprüche 1 bis 5, das nicht glykosyliert ist.

7. Strukturgen mit einer DNA-Sequenz, die ein synthetisches Mutein nach einem der Ansprüche 1 bis 6 codiert.

8. Expressionsvektor, der ein Strukturgen nach Anspruch 7 enthält.

9. Plasmid pSY2501, das aus dem unter der Hinterlegungs-Nr. NRRL B-15356 hinterlegten E. coli-Stamm erhältlich ist.

10. E. coli, das mit einem Expressions-Vektor nach Anspruch 8 oder mit einem Plasmid nach Anspruch 9 transformiert ist.

11. Verwendung eines Expressionsvektors nach Anspruch 8 oder eines Plasmids nach Anspruch 9 bei der Herstellung eines synthetischen rekombinanten, menschlichen β-Interferon-Muteins durch Expression des Vektors oder des Plasmids.

12. Verfahren zur Verhinderung der Bildung einer falschen Disulfidbindung durch ein menschliches β-Interferon-Protein, das mindestens einen für die Bildung dieser Bindung verfügbaren Cystein-Rest aufweist, bei dem man das Protein durch Deletion des entsprechend der Numerierung von natürlichem menschlichem β-Interferon an Position 17 befindlichen Cystein-Rests durch Mutation verändert, oder den Cystein-Rest durch eine neutrale Aminosäure ersetzt.

13. Verfahren nach Anspruch 12, in dem der Cystein-Rest durch Serin oder Threonin ersetzt wird.

14. Verfahren zur Herstellung eines Strukturgens nach Anspruch 7, bei dem man folgende Schritte durchführt:

(a) Hybridisierung einzelsträngiger DNA, die einen Strang eines Strukturgens enthält, das für ein menschliches β-Interferon-Protein codiert, mit einem mutierten Oligonucleotid-Primer, der zu einem Bereich des Stranges komplementär ist, der das Codon für den Cystein-Rest oder, je nach dem das mit dem Codon gepaarte Antisens-Triplett enthält, mit Ausnahme einer Fehlpaarung mit dem Codon oder dem Antisens-Triplett, die eine Deletion des Codons oder ein Triplett definiert, das für die neutrale Aminosäure codiert;

(b) Verlängerung des Primers mit DNA-Polymerase zur Bildung einer mutierten Heteroduplex-Struktur; und

(c) Replikation der mutierten Heteroduplex-Struktur.

15. Verfahren nach Anspruch 14, bei dem die Fehlpaarung ein Triplett definiert, das für Serin oder Threonin codiert.

16. Verfahren nach Anspruch 14 oder 15, bei dem die einzelsträngige DNA ein den Strang enthaltender einzelsträngiger Phage ist und die mutierte Heteroduplex-Struktur aus Schritt (b) in ein geschlossene, ringförmige Heteroduplex-Struktur überführt wird.

17. Verfahren nach einem der Ansprüche 14 bis 16, bei dem die Replikation durch Transformation eines kompetenten bakteriellen Wirtes mit der geschlossenen, ringförmigen Heteroduplex-Struktur und die Züchtung der erhaltenen Transformante bewirkt wird.

18. Verfahren nach einem der Ansprüche 14 bis 17, das die weiteren Schritte der Isolierung der Nachkommen des mutierten Stranges der Heteroduplex-Struktur, die Isolierung von DNA aus den Nachkommen und die Isolierung des Gens aus der DNA der Nachkommen umfaßt.

19. Verfahren nach einem der Ansprüche 14 bis 18, bei dem die Fehlpaarung ein für Serin codierendes Codon definiert.

20. Oligonucleotid zur Verwendung bei der Herstellung eines Strukturgens nach Anspruch 7, durch Oligonucleotid-gesteuerte Mutagenese, das eine Nucleotid-Sequenz aufweist, die komplementär zur einem Bereich des Stranges des Strukturgens ist, der das Codon für den Cystein-Rest oder, je nach dem, das mit dem Codon gepaarte Antisens-Triplett enthält, mit Ausnahme einer Fehlpaarung mit dem Codon, die ein Triplett definiert, das eine neutrale Aminosäure codiert.

21. Verfahren zur Herstellung eines Oligonucleotids, das bei der Herstellung eines Strukturgens nach Anspruch 7 durch Oligonucleotid-gesteuerte Mutagenese verwendbar ist, bei dem man in an sich bekannter Weise eine Nucleotid-Sequenz synthetisiert, die komplementär zu einem Bereich des Stranges des Strukturgens ist, der das Codon für den Cystein-Rest oder, je nach dem, das mit dem Codon gepaarte Antisens-Triplett enthält, mit Ausnahme einer Fehlpaarung mit dem Codon, die eine Deletion des Codons oder ein Triplett definiert, das für eine neutrale Aminosäure codiert.

22. Verfahren zur Herstellung eines Vektors nach Anspruch 8, bei dem man in einen zur Transformation einer Wirtszelle geeigneten Vektor ein Gen nach Anspruch 7 in einer Stellung inseriert, die die Expression des Gens gestattet.

23. Pharmaceutische oder tiermedizidinische Formulierung mit menschlicher β-Interferon-Aktivität, die ein synthetisches Mutein-Präparat nach einem der Ansprüche 1 bis 6 enthält, das zur pharmazeutischen bzw. zur tiermedizinischen Verwendung formuliert ist, und die gegebenenfalls außerdem ein pharmazeutische oder tiermedizinisch verträgliches Verdünnungsmittel, einen Träger oder Exzipienz enthält, und/oder die gegebenenfalls als Einheitsdosierung vorliegt.

24. Formulierung zur Verwendung bei der Krebs-Therapie, enthaltend:

(a) eine wirksame Menge eines rekombinanten, meschlichen β-Interferon-Muteins, in dem der entsprechend der Numerierung von natürlichem menschlichem β-Interferon an Position 17 befindliche Cystein-Rest deletiert oder durch eine andere Aminosäure ersetzt ist, wobei das Mutein die biologische Aktivität von natürlichem, menschlichem β-Interferon aufweist; und

(b) ein nicht-toxisches, nicht-allergenes, therapeutisch vertägliches Trägermaterial.

25. Formulierung nach Anspruch 24, in der das Mutein ein menschliches Serin$_{17}$-β-Interferon ist und der Träger destilliertes Wasser, Ringer-Lösung, Hank-Lösung oder physiologische Kochsalzlösung ist.

26. Formuliertung nach Anspruch 24 oder 25, die nicht-toxische, stabilisierende und löslichmachende Zusatzstoffe enthält, die ausgewählt sind aus Dextrose und menschlichem Serumalbumin.

27. Formulierung nach einem der Ansprüche 24 bis 26 als Einheitsdosierung.

28. Formulierung nach einem der Ansprüche 24 bis 26 in örtlich applizierbarer Form.

29. Synthetisches Mutein nach einem der Ansprüche 1 bis 6 zur Verwendung bei der Prophylaxe, Therapie oder Diagnose, durchgeführt am menschlichen oder tierischen Körper.

30. Verfahren zur Herstellung einer pharmazeutischen oder tiermedizinischen Formulierung nach Anspruch 23, bei dem man ein synthetisches Mutein nach einem der Ansprüche 1 bis 6 zur pharmazeutischen oder tiermedizinischen Verwendung formuliert.

**Revendications**

1. Mutéine d'interféron bêta humain synthétique recombinante, biologiquement active, dans laquelle le reste cystéine normalement présent en position 17, numéroté conformément à l'interféron bêta humain natif, et qui est susceptible de former une liaison disulfure et n'est pas essentiel pour ladite activité biologique, a été supprimé ou remplacé par un aminoacide neutre.

2. Mutiéine selon la revendication 1, dans laquelle ladite cystéine est remplacée par la sérine, la thréonine, la glycine, l'alanine, la valine, la leucine, l'isoleucine, l'histidine, la tyrosine, la phénylalanine, le tryptophane ou la méthionine.

3. Mutéine selon la revendication 1, dans laquelle ledit aminoacide neutre est la sérine.

4. Mutéine d'interféron bêta sérin$_{17}$ recombinante humaine.

5. Mutéine d'interféron bêta sérin$_{17}$ recombinante humaine, manifestant l'activité biologique de l'interféron bêta humain natif et ayant la séquence d'aminoacides déduite telle que représentée sur la figure 10, avec ou sans méthionine N-terminale.

6. Mutéine l'une quelconque des revendications 1 à 5 et qui est non glycosylée.

7. Gène structural comportant une séquence d'ADN qui code pour une mutéine synthétique telle que définie dans l'une quelconque des revendications 1 à 6.

8. Vecteur d'expression comprenant un gène structural tel que défini dans la revendication 7.

9. Plasmide pSY2501, tel que pouvant être obtenu à partir de la souche de *E. coli* déposée sous le n° NRRL B-15356.

10. *E. coli* transformé par un vecteur d'expression tel que défini dans la revendication 8 ou par un plasmide tel que défini dans la revendication 9.

11. Utilisation d'un vecteur d'expression tel que défini dans la revendication 8 ou d'un plasmide tel que défini dans la revendication 9, dans la préparation d'une mutéine d'interféron bêta humain recombinante synthétique, par expression dudit vecteur ou plasmide.

12. Procédé d'empêchement à une protéine de type interféron bêta humain, comportant au moins un reste cystéine susceptible de former une liaison disulfure incorrecte, de former ladite liaison, comprenant l'altération mutationnelle de la protéine par délétion du reste cystéine en position 17, numéroté conformément à l'interféron bêta humain natif, ou remplacement dudit reste cystéine par un aminoacide neutre.

13. Procédé selon la revendication 12, dans lequel le reste cystéine est remplacé par la sérine ou la thréonine.

14. Procédé pour la préparation d'un gène structural tel que défini dans la revendication 7, comprenant:

(a) l'hybridation d'ADN monocaténaire comprenant un brin d'un gène structural codant pour une protéine de type interféron bêta humain, avec une amorce oligonucléotidique mutante qui est complémentaire d'une région dudit brin contenant le codon codant pour ledit reste cystéine ou le triplet antisens apparié dudit codon, selon le cas, à l'exception d'un mésappariement avec ledit codon ou ledit triplet antisens, lequel mésappariement définit une délétion du codon ou un triplet codant pour ledit aminoacide neutre;

(b) l'extension de l'amorce à l'aide d'ADN polymérase, pour former un hétéroduplex mutationnel; et

(c) la réplication dudit hétéroduplex mutationnel.

15. Procédé selon la revendication 14, dans lequel le mésappariement définit un triplet codant pour la sérine ou la thréonine.

16. Procédé selon la revendication 14, ou la revendication 15, dans lequel l'ADN monocaténaire est un phage monocaténaire qui comprend ledit brin, et l'hétéroduplex mutationnel de l'étape (b) est converti en un hétéroduplex circulaire fermé.

17. Procédé selon l'une quelconque des revendications 14 à 16, dans lequel ladite réplication est effectuée par transformation d'un hôte bactérien compétent par l'hétéroduplex circulaire fermé, et la culture des transformants résultants.

18. Procédé selon l'une quelconque des revendications 14 à 17 et comprenant les étapes supplémentaires d'isolement de descendance du brin mutant de l'hétéroduplex, l'isolement d'ADN à partir de ladite descendance et l'isolement dudit gène à partir de l'ADN provenant de ladite descendance.

19. Procédé selon l'une quelconque des revendications 14 à 18 dans lequel le mésappariement définit un codon codant pour la sérine.

20. Oligonucléodide à utiliser dans la préparation d'un gène structural tel que défini dans la revendication 7, par mutagénèse dirigée sur oligonucléotide, et comportant une séquence de nucléotides qui est complémentaire d'une région du brin du gène structural qui comprend le codon codant pour le reste cystéine ou le triplet antisens apparié audit codon, selon le cas, à l'exception d'un mésappariement avec ledit codon, lequel définit un triplet codant pour ledit aminoacide neutre.

21. Procédé pour la préparation d'un oligonucléotide approprié à l'utilisation dans la production d'un gène structural tel que défini dans la revendication 7, par mutagénèse dirigée sur oligonucléotide, lequel comprend la synthèse, d'une façon connue en soi, d'une séquence nucléotidique complémentaire d'une région du brin du gène structural comprenant le codon codant pour le reste cystéine ou le triplet antisens apparié audit codon, selon le cas, à l'exception d'un mésappariement avec ledit codon, lequel définit une délétion du codon ou un triplet codant pour un aminoacide neutre.

22. Procédé pour la préparation d'un vecteur tel que défini dans la revendication 8, comprenant l'insertion dans un vecteur capable de transformer une cellule hôte, d'un gène tel que défini dans la revendication 7, en une position permettant l'expression dudit gène.

23. Composition pharmaceutique ou vétérinaire ayant une activité d'interféron bêta humain, comprenant une préparation de mutéine synthétique selon l'une quelconque des revendications 1 à 6, formulée pour l'utilisation pharmaceutique ou vétérinaire, respectivement, et éventuellement comprenant aussi un diluant, véhicule ou excipient à usage pharmaceutique ou vétérinaire et/ou se trouvant éventuellement sous forme de dose unitaire.

24. Composition à utiliser dans le traitement de cancers, comprenant:

(a) une quantité efficace, d'une mutéine d'interféron bêta humain recombinante, dans laquelle le reste cystéine en position 17, numéroté conformément à l'interféron bêta humain natif, est supprimé ou remplacé par une autre aminoacide, et ladite mutéine manifeste l'activité biologique de l'interféron bêta humain natif; et

(b) un véhicule non toxique, non allergène, thérapeutiquement acceptable.

25. Composition selon la revendication 24, dans laquelle la mutéine est l'interféron bêta-sérine$_{17}$ humain, et ledit véhicule est l'eau distillée, la solution de Ringer, la solution de Hank ou le sérum physiologique.

26. Composition selon la revendication 24 ou 25, comprenant des additifs à effet stabilisant et solubilisant non toxiques, choisis parmi le D-glucose et l'albumine de sérum humain.

27. Composition selon l'une quelconque des revendications 24 à 26, et sous forme de dose unitaire.

28. Composition selon l'une quelconque des revendications 24 à 26, et sous forme destinée à l'application locale.

29. Mutéine synthétique selon l'une quelconque des revendication 1 à 6, utiliser dans la prophylaxie, le traitement ou le diagnostic pratiqués sur l'organisme humain ou animal.

30. Procédé pour la préparation d'une composition pharmaceutique ou vétérinaire telle que définie dans la revendication 23, lequel procédé comprend la formulation à usage pharmaceutique ou vétérinaire, respectivement, d'une mutèine synthétique telle que définie dans l'une quelconque des revendications 1 à 6.

```
           5               10              15              20
MetSerTyrAsnLeu LeuGlyPheLeuGln ArgSerSerAsnPhe GlnCysGlnLysLeu
          25               30              35              40
LeuTrpGlnLeuAsn GlyArgLeuGluTyr CysLeuLysAspArg MetAsnPheAspIle
          45               50              55              60
ProGluGluIleLys GlnLeuGlnGlnPhe GlnLysGluAspAla AlaLeuThrIleTyr
          65               70              75              80
GluMetLeuGlnAsn IlePheAlaIlePhe ArgGlnAspSerSer SerThrGlyTrpAsn
          85               90              95             100
GluThrIleValGlu AsnLeuLeuAlaAsn ValTyrHisGlnIle AsnHisLeuLysThr
         105              110             115             120
ValLeuGluGluLys LeuGluLysGluAsp PheThrArgGlyLys LeuMetSerSerLeu
         125              130             135             140
HisLeuLysArgTyr TyrGlyArgIleLeu HisTyrLeuLysAla LysGluTyrSerHis
         145              150             155             160
CysAlaTrpThrIle ValArgValGluIle LeuAgAsnPheTyr PheIleAsnArgLeu
         165              170             175             180
ThrGlyTyrLeuArg Asn---
```

# FIG. I

FIG. 2

FIG. 3

FIG. 4

FIG. 5

FIG. 7

EP 0 218 825 B1

FIG. 6

5

EP 0 218 825 B1

FIG. 8a

pβ1trp

pSY2501

FIG. 8b

6

FIG. 9

IFN-B CYS TO SER CHANGE AT AMINO ACID 17

1                                                                                    17
ATG AGC TAC AAC TTG CTT GGA TTC CTA CAA AGA AGC AGC AAT TTT CAG AGT CAG AAG CTC
met ser tyr asn leu leu gly phe leu gln arg ser ser asn phe gln ser gln lys leu

61
CTG TGG CAA TTG AAT GGG AGG CTT GAA TAT TGC CTC AAG GAC AGG ATG AAC TTT GAC ATC
leu trp gln leu asn gly arg leu glu tyr cys leu lys asp arg met asn phe asp ile

121
CCT GAG GAG ATT AAG CAG CTG CAG CAG TTC CAG AAG GAG GAC GCC GCA TTG ACC ATC TAT
pro glu glu ile lys gln leu gln gln phe gln lys glu asp ala ala leu thr ile tyr

181
GAG ATG CTC CAG AAC ATC TTT GCT ATT TTC AGA CAA GAT TCA TCT AGC ACT GGC TGG AAT
glu met leu gln asn ile phe ala ile phe arg gln asp ser ser ser thr gly trp asn

241
GAG ACT ATT GTT GAG AAC CTC CTG GCT AAT GTC TAT CAT CAG ATA AAC CAT CTG AAG ACA
glu thr ile val glu asn leu leu ala asn val tyr his gln ile asn his leu lys thr

301
GTC CTG GAA GAA AAA CTG GAG AAA GAA GAT TTC ACC AGG GGA AAA CTC ATG AGC AGT CTG
val leu glu glu lys leu glu lys glu asp phe thr arg gly lys leu met ser ser leu

361
CAC CTG AAA AGA TAT TAT GGG AGG ATT CTG CAT TAC CTG AAG GCC AAG GAG TAC AGT CAC
his leu lys arg tyr tyr gly arg ile leu his tyr leu lys ala lys glu tyr ser his

421
TGT GCC TGG ACC ATA GTC AGA GTG GAA ATC CTA AGG AAC TTT TAC TTC ATT AAC AGA CTT
cys ala trp thr ile val arg val glu ile leu arg asn phe tyr phe ile asn arg leu

481
ACA GGT TAC CTC CGA AAC TGA AGA TC
thr gly tyr leu arg asn ***

# FIG. 10

FIG. II